(19)

**Europäisches Patentamt**
**European Patent Office**
**Office européen des brevets**

(11) **EP 4 685 803 A1**

(12) **EUROPEAN PATENT APPLICATION**

(43) Date of publication:
**28.01.2026 Bulletin 2026/05**

(21) Application number: **24190466.3**

(22) Date of filing: **23.07.2024**

(51) International Patent Classification (IPC):
**G16B 40/10** (2019.01)    **G01N 33/68** (2006.01)
**G16B 40/20** (2019.01)

(52) Cooperative Patent Classification (CPC):
**G16B 40/10; G01N 33/6848; G16B 40/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Oxford University Innovation Limited
Oxford, OX2 0JB (GB)**

(72) Inventors:
• **BENNETT, Jack**
  **Oxford, OX2 0JB (GB)**
• **EL-BABA, Tarick**
  **Oxford, OX2 0JB (GB)**
• **LUTOMSKI, Corrinne**
  **Oxford, OX2 0JB (GB)**
• **ROBINSON, Carol**
  **Oxford, OX2 0JB (GB)**

(74) Representative: **HGF
HGF Limited
4th Floor, 1 City Square
Leeds LS1 2AL (GB)**

(54) **IMPROVED MASS SPECTROMETRY METHODS**

(57) The invention relates to computer implemented methods of identifying the presence of one or more modification(s) of a protein; and removing false fragments from a list of fragments generated by one or more mass deconvolution algorithm(s). Also provided are one or more computer program storage media or computer programs which, when executed by a computer, cause the computer to carry out the methods; and apparatus configured to carry out the methods. Also provided is a method for generating a trained model to detect false fragments from a list of fragments generated by one or more mass deconvolution algorithm(s) and the resulting trained model.

Figure 1

**Description**

**Field of the Invention**

The invention relates to computer implemented methods of identifying the presence of one or more modification(s) of a protein; and removing false fragments from a list of fragments generated by one or more mass deconvolution algorithm(s). Also provided are one or more computer program storage media or computer programs which, when executed by a computer, cause the computer to carry out the methods; and apparatus configured to carry out the methods. Also provided is a method for generating a trained model to detect false fragments from a list of fragments generated by one or more mass deconvolution algorithm(s) and the resulting trained model.

**Background**

Post-translational modifications (PTMs) precisely regulate biomolecular function by modulating protein dynamics, interactions, and localisation patterns. Distinctly modified protein isoforms (i.e. proteoforms) act as unique biological effectors, enabling tight and dynamic control of cellular phenotypes.

Mass spectrometry (MS)-based proteomics has been used to correlate specific proteoforms with unique cellular states by identifying and quantifying modified proteins. However, it remains challenging to identify the direct effect of such modifications on protein structure and consequently their significance for molecular function.

Native and intact protein mass spectra which may also contain post-translational modifications are often difficult to interpret due to their complexity.

**Summary of the Invention**

The inventors have addressed this problem by designing an analytical workflow for the discovery of protein modifications from top-down mass spectra. This workflow includes several novel methods each of which (or can be used together in the workflow) optimise accurate data gathering from these type of spectrum data.

Therefore, in a first aspect of the invention, there is provided a computer-implemented method of identifying the presence of one or more modification(s) of a protein, the method comprising:

> a) obtaining deconvoluted product ion mass spectrum data comprising masses of fragment ions produced by activating intact protein ions;
> b) applying a mass offset to a plurality of theoretical terminal fragment ion masses calculated from the amino acid sequence of the protein, and matching the masses including the offset to the list of observed masses in the deconvoluted product ion mass spectrum data; and
> c) if a plurality of matches are identified in b), identifying the presence of a modification with mass equal to that of the mass offset in a plurality of terminal fragment ions originating from the protein, optionally further comprising
> d) identifying fragments comprising the modification(s) from the plurality of terminal fragment ions in c); and/or identifying the modification(s).

In a second or further aspect of the invention, there is provided a computer-implemented method of removing one or more false fragments from a list of fragments generated by one or more mass deconvolution algorithm(s) applied to a product ion mass spectrum produced by activating intact protein ions, the method comprising:

> a) inputting isotopic envelope properties calculated for one or more fragments in a deconvoluted product ion mass spectrum from an intact protein ion into a plurality of trained machine learning models, the plurality of trained machine learning models trained to distinguish between false and true fragments in a list of fragments obtained from the spectrum;
> b) identifying one or more fragments as false if identified as false by at least two trained machine learning models; and
> c) removing the fragment(s) identified as false in b) from the list of fragments.

In a further aspect of the second aspect of the invention there is provided a computer-implemented method for generating a trained model to detect false fragments from a list of fragments generated by one or more mass deconvolution algorithm(s) applied to a product ion mass spectrum obtained from an intact, optionally a native protein sample, the method comprising:

> a) obtaining measurements from deconvoluted intact or native protein product ion mass spectra, optionally wherein the measurements comprise any one or more of the following:

i) the fragment ion charge; ii) a goodness-of-fit score that measures the similarity of the observed fragment envelope to that of a typical protein fragment ion envelope; iii) the proportion of signal within the fragment ion envelope's span that can be attributed to the fragment ion itself; iv) the signal-to-noise ratio; v) the number of missing isotopologues; vi) the individual isotopologues mass errors; and vii) the Pearson correlation coefficient calculated by correlating the observed isotopologues intensities with those expected for a typical protein fragment ion;

b) obtaining ground truth data for each of the fragment(s) indicating if the fragment(s) is a true or false fragment; and
c) training a machine learning algorithm to relate a) with b) to produce a trained model, wherein the algorithm is trained to maximize recall.

In a further aspect of the second aspect of the invention there is provided a computer-implemented trained model obtained from the above method.

In further aspects of the inventions there is provided one or more computer-readable storage media or a computer program comprising instructions which, when executed by a computer, cause the computer to carry out the above methods.

In a further aspect of the first and/or second aspects of the invention, there is provided an apparatus comprising:

a)

i) a memory storing 2 or more trained machine learning models; and
ii) processing circuitry communicatively coupled to the memory, the processing circuitry configured to:

run the trained machine learning models to receive as input isotopic envelope properties calculated for one or more fragments in a deconvoluted product ion mass spectrum obtained from an intact, optionally native, protein sample;
identify one or more fragment(s) in a list from the spectrum as false if identified as false by at least two of the trained machine learning models; and
remove the fragment(s) identified as false from the list of fragments to output a filtered list of fragments;

and/or

b)

i) processing circuitry;
ii) a memory communicatively coupled to the processing circuitry, the memory storing instructions executable by the processing circuitry for applying a mass offset to a plurality of theoretical terminal fragment ion masses calculated from the amino acid sequence of a protein, and matching the masses including the offset the list of observed masses in deconvoluted product ion mass spectrum data; identifying if there are a plurality of matches identified; and if a plurality of matches are identified, identifying the presence of a modification with mass equal to the mass offset in a plurality of terminal fragment ions originating from a protein;
the processing circuitry configured to:

execute the instructions to receive as input deconvoluted product ion mass spectrum data comprising masses of fragment ions produced by activating an ion of the intact, optionally native protein, optionally wherein the data is the filtered list of fragment envelopes output from a); and
output an indication of the presence of a modification with mass equal to the mass offset in a plurality of terminal fragment ions originating from the protein; and optionally additionally output: the identity of one or more fragments of the protein which comprise the modification and/or the identity the modification.

**Detailed description**

***General***

*Intact and native protein mass spectrum and mass spectrum data*

By intact, it is meant that the protein is not proteolyzed in the sample (for example, using trypsin, i.e. digested by a protease) prior to mass spectrometry analyses. That is, the full-length protein is converted into a gas phase ion for mass spectrometric analysis. The intact mass spectrum may be measured under native conditions, i.e. where the buffer is at a pH and ionic strength that maintains the protein's native structure in solution. In native mass spectrometry, the objective is to transfer the analytes, predominantly intact proteins and protein complexes, into the mass analyzer while retaining their original solution-phase inter- and intra-molecular interactions and structure. Therefore, for intact samples the protein is the full-length, undigested sample. For native samples, the protein is the full-length, undigested (i.e. not proteolyzed, backbone intact) sample in conditions which maintain the secondary, tertiary and/or quaternary structure of the protein. By activating intact protein ions is meant activating (or fragmenting) protein ions in a sample where the protein is not digested as explained above. By activating native protein ions is meant activating or fragmenting protein ions in a sample where the protein is not proteolyzed and the protein is in a solution which maintains the protein's secondary, tertiary structure and/or quaternary structure.

*Product ion mass spectrum data*

A product ion mass spectrum is measured by $m/z$-selecting precursor ions (intact protein ions), subjecting these selected ions to an ion transformation process to form product ions, and measuring the mass-to-charge ratio of the product ions. The data from this first process are spectra containing lists of ions with distinct mass-to-charge ratios and the relative quantities of each of these ions. Deconvolution algorithms are then used to analyse these spectra and detect groups of ions/peaks (called envelopes) that correspond with distinct molecular species. Then, the output of the deconvolution algorithm is a list of monoisotopic masses of product ions. This list of masses is the data input into the method of identifying the presence of one or more modification(s) of an amino acid containing fragment of a protein. The intact protein ions are protein ions from intact protein samples (i.e. undigested protein samples). Optionally, the protein ions may be from native protein samples, i.e. in conditions to keep the secondary structure, tertiary structure and/or quaternary structure of the protein intact.

Product ions are formed via an ion transformation process (e.g. fragmentation). Therefore, the product ion mass spectrum data may be obtained by subjecting an intact protein ion to an ion transformation process as described here to generate fragment ions.

*Recalibration*

Recalibration of the observed ion masses allows the use of smaller mass tolerances in searches which in turn increases the significance of the matches.

Recalibration may be carried out before step b) of claim 1. That is, theoretical fragment ion masses are calculated based on only the amino acid sequence of the protein and these are matched with observed peaks in the spectrum. The observed spectrum is then recalibrated based on adjusting the observed spectrum to align with the theoretical ion masses.

For the method of removing false fragments, recalibration may be performed after filtering the product ion mass spectrum to remove false envelopes.

Where the method involves applying the method of filtering to remove false envelopes and then using this filtered set of envelopes to identify the presence of a modification, recalibration may be undertaken after performing the filtering steps, specifically after calculating the theoretical fragment ion masses. These are then matched up with the filtered envelope masses and the list of peaks updated based on matching the filtered envelope masses with the theoretical fragment ion masses.

*Mass Accuracy*

Mass accuracy is the deviation of the measured mass from the true mass. The level of mass accuracy can be determined by reference to the mass error, reported as either an absolute error (measured mass less true mass) or a relative error (absolute mass error divided by true mass). The errors are typically reported in millidalton (mDa) or microdalton (μDa) for absolute mass errors and as parts per million (ppm) or parts per billion (ppb) for relative mass errors.

*Ion transformation process*

Ions may be subject to different ion transformation processes in a mass spectrometer.

Ion transformation processes can involve fragmentation (e.g. of precursor ions) via the introduction of energy to break one or more covalent and/or non-covalent bonds (i.e. fragmentation). Non-limiting examples of ion transformation processes that can involve fragmentation include any one or more of the following: photon induced dissociation (such as infrared multiphoton dissociation, blackbody infrared radiative dissociation, and/or ultraviolet photodissociation), electron capture and transfer methods (electron transfer dissociation, activated ion electron transfer dissociation, electron capture dissociation, and/or electron-induced dissociation), surface-induced dissociation, and/or collision-induced dissociation.

Ion transformation processes can involve a non-dissociative process. A non-limiting example of a (typically) non-dissociative process is charge modulation. Charge modulation may be provided by ion-ion or ion-molecule reactions.

Ion transformation processes may involve a combination of more than one ion transformation process. For example, ions may be subject to a non-dissociative process (such as charge modulation) followed by a process that involves fragmentation. In addition, where a mass spectrum is obtained after more than one stage of $m/z$ separation, ions may be subject to at least one ion transformation process before each subsequent stage of $m/z$ separation; and ions may optionally be subject to at least one ion transformation process before the first stage of $m/z$ separation.

### Types of mass spectrum

The mass spectrum may be a standard mass spectrum or a product ion mass spectrum. In a standard spectrum, a $m/z$ separation and detection is applied to record ions generated by the ion source. In a product ion spectrum, the precursor ion(s) are selected by an initial stage of $m/z$ separation, the selected ions are subjected to an ion transformation process (e.g. fragmentation), and a subsequent stage of $m/z$ separation and detection is applied to the product ions.

Selection of precursor ions may involve spatial and/or temporal separation of the precursor ions from other ions on the basis of $m/z$.

$m/z$ separation and detection may involve spatial separation, and/or temporal separation, and/or frequency separation of ions on the basis of their $m/z$. For example, a time-of-flight mass analyser provides temporal separation, while an Orbitrap or Fourier-transform ion cyclotron resonance (FT-ICR) mass analyzer provides frequency separation.

As the skilled person will appreciate, an $n^{th}$ generation product ion spectrum may be obtained by using $m/z$ separation to select a product ion (of the $n^{th}$-1 generation) for dissociation, with the further generation of product ions ($n^{th}$ generation) subject to mass analysis.

A product ion spectrum may also be obtained where an ion transformation process is performed without initial $m/z$ separation. Where the ion transformation process involves fragmentation without initial $m/z$ separation, this provides a pseudo-MS/MS (pMS$^2$) spectrum; or a pMS" spectrum.

### Method of identifying modifications

The inventors have identified a method which detects modified fragment ions formed from intact protein ions. The method does not require a knowledge of the intact mass of the protein or predefined hypotheses regarding the identity of the modifications. This is especially useful when the intact protein mass spectrum is complicated or "messy", and/or the resolution of the intact protein mass spectrum is not sufficient to resolve individual isotopologues and/or intact protein ions with overlapping molecular mass distributions. This is the case for many native mass spectrometry measurements and also in denaturing, intact mass spectrometry where, for example, the protein is heterogeneously modified.

### Fragment and product ions

A product ion is the charged product of an ion transformation process. Fragment ions are a specific class of product ions that are formed via the breakage of one or more covalent and/or non-covalent bonds. A product ion may be stable over the timescale of mass analysis or may dissociate further to form other charged fragment ions and neutral species of successively lower mass prior to mass analysis.

Protein product ions can either be:

(i) a terminal fragment ion, where only a single cleavage event occurs to generate N-terminal-containing *a, b, c,* fragments and/or C-terminal-containing *x, y,* z fragments

or

(ii) an internal fragment ion, where two cleavage events occur

Generating for example *ax, ay, az, bx, by, bz, cx, cy, cz* fragment ions depending on the activation method used.

Therefore, the mass offset may be applied to any of fragments *a, b,* or *c*; and/or x, *y* or z. The set of terminal fragment ions originating from the protein which are identified as having the modification may therefore be any of fragments *a, b,* or *c*; and/or *x, y* or *z.*

The internal fragment ions identified may comprise for example, any of *ax*, *ay*, *az*, *bx*, *by*, *ex,* cy and/or cz ions.

### Protein ion

A protein ion is produced by converting a protein molecule into a gas-phase ion using an ion source.

*Modification*

The modification may be any covalent or non-covalent modification to an amino acid fragment. That is, the modification is a covalent or non-covalent ligand bound to the amino acid fragment. For example, the modification may be any one or more of: post-translational modifications; co-translational modifications; chemical modifications; small molecule or metal ion binding; and/or gas-phase modifications. For example, the post-translational modifications may be proteolysis, glycosylation, phosphorylation, and/or lipidation.

For example, the non-covalently or covalently bound ligands may be therapeutic agents (drugs), metabolites, metal ions and/or lipids.

The modification may also be a mutation, i.e. a change from the canonical sequence. In this case the theoretical fragment ion mass is calculated from the canonical amino acid sequence. The offset is the mutation (i.e. the difference in mass between the canonical amino acid in the fragment and the mutation).

The modification can also be a gas-phase modification of a terminal fragment to produce an internal fragment ion. In this case, the modification is the loss of a peptide fragment ion from the terminal fragment to produce an ion that does not contain either of the intact protein's termini.

*Calculating the theoretical fragment ion masses from the amino acid sequence of the protein*

The theoretical ion masses are based on the amino acid sequence of the protein and any modifications to the protein's termini. For example, a protein's N-terminus can be modified with an acetyl group.

Masses for fragments of the protein can be calculated. For example, for b- and y-type fragments produced by collision-induced dissociation.

For a b-type ion the mass of a theoretical fragment is equal to the mass of the N-terminal modification plus the fragment sequence mass (where the fragment sequence mass is calculated as the sum of the masses of the dehydrated amino acid residues that comprise the fragment).

For a y-type ion the mass of a theoretical fragment is equal to the mass of the C-terminal modification plus the fragment sequence mass plus the mass of water.

That is, the theoretical fragment ion masses are calculated, residue by residue from the N and C terminus to determine the theoretical terminal fragment ion masses. The result is a plurality of theoretical masses from either the N terminus and/or the C terminus. For example, starting from the N terminus: AA1-2; AA1-3; AA1-4 and so on, for all the b ions.

*Mass offset*

The mass offset is a mass added to the theoretical fragment ion masses calculated from the amino acid sequence of the protein. The resulting theoretical fragment ion masses including the mass offset can then be matched to the list of observed fragment masses. If a plurality of matches are observed, then this indicates that there are modified fragments in the product ion mass spectrum with modification(s) that has mass equal to that of the mass offset.

The mass offset can be scanned across a range of potential mass offsets using a sliding window approach to afford an offset scan. The significance of each peak in the resulting offset scan can be calculated. That is, once a plurality of matches has been identified in step b) of claim 1, the likelihood that this number of matches or greater would be observed if none of the fragment ions in the spectrum were modified can be calculated. This statistical approach accounts for random matches at a given offset that do not arise from true correspondence (s) between the offset theoretical fragment ions and the observed fragment ions. This is described in more detail below.

This can be done by estimating a mathematical distribution that describes the number of matches that could be expected if none of the fragment ions were modified. Preferably, this distribution is a Poisson distribution with an expected value equal to the average (mean) number of matches observed across the full offset scan. Other distributions could also be used, for example the distribution could be calculated by simulation. The Poisson distribution can be used to calculate an expectation-value (e-value) which is the expected number of times that the observed number of matches or greater would be observed for an offset, upon evaluating *n* offsets (explained in Lynn et al, 2006, 54th ASMS Conference on Mass Spectrometry: "Protein Prospector and Ways of Calculating Expectation Values". It can be expressed as

$$E - value \ = \ \frac{\lambda^k e^{-\lambda}}{k!} \times \mathrm{n}$$

where k is the number of matches for a given offset, lambda is the expected value, and n is the number of offsets evaluated.

The offset is a non-zero offset. For example, a range of values may be used as an offset that encompass most common modifications, e.g. approximately -150 to 1500 Da, for example, -150 to 500 Da. The offset may be scanned across this range of masses in a sliding window manner, where the window is shifted by approximately 50% of the mass tolerance used for matching. The number of matches is calculated for each window.

The plurality of terminal fragment ions originating from the protein with the offset may be from one end of the terminus of the protein (e.g. plurality of b ions, or alternatively a plurality of y ions). Alternatively, the plurality of terminal fragment ions with the offset may be from both ends of the protein, for example where the modification is bound to an amino acid in the middle of the protein one can imagine a b ion containing the offset and a y ion containing the offset.

Once a plurality of fragments with the offset are identified, the identity of the modification can then be determined based on its exact mass. Various methods are available in the art, for example, the offsets can be searched against UniMod, a database which contains the masses of most common fragment ion modifications.

The mass of the modification identified is equal to that of the mass offset. This is not necessarily always the mass of the original protein modification. For example, for glycosylated proteins, the original modification may be Man5GlcNAc2 (1216 Da) but only fragment ions with the offset of GlcNAc (203 Da) are observed in the product ion mass spectrum. This is because some protein modifications may partially decompose or rearrange during activation so that only a portion of them remains on the fragment ion.

### Identifying the fragments comprising the modification(s)

Once you have identified an offset with a plurality of matches, all ions in the spectrum that comprise the set of matched ions can be assigned as modified terminal fragment ions.

This can be achieved by matching the list of offset theoretical ions to the observed fragments list. Observed fragments that match are assigned and the amino acid sequence of the modified fragment ions is obtained.

### Removing peaks (masses in the data)

Peaks matching calculated masses based on only the amino acid residues of the fragments are removed. Additionally, other easily recognisable masses belonging to peaks matching dehydrated, deamidated and/or alkali metal ions of these calculated masses can be removed from the peak list. This is useful as it leaves only the unassigned peaks. These peaks can be calculated as having the following additional masses: Dehydration (-18.010565); Deamidation (-17.026549), Sodium adduction (+21.981945); Lithium adduction (+6.00818); and Potassium adduction (+37.955883). Note the loss of one proton in the case of charged adducts.

### Determining the amino acid sequence of the modified fragments and optionally localizing the modification

Once the fragments with the modification have been identified, the amino acid sequence can be obtained for the matched fragments by comparing the observed mass with the calculated (theoretical mass) of the terminal fragment ion plus the offset.

In addition, the position of the modification in the protein sequence can be obtained. This can be done by detecting any overlapping modified fragments. The modification must be positioned within the span of residues over which the modified fragments overlap. For example, where the ions are b ions, the modification can be localised to the overlapping b ions identified as containing the modification (matched). Alternatively, where the modification is located on an amino acid in the middle of the protein, it may be overlapping b and y ions which allow localisation of the modification (when both ions are observed as containing the offset, therefore the modification must be attached to an amino acid where the modified b and y ions overlap).

Optionally, the modification can be further localised, for product ion mass spectrum data where the overlapping fragments do not definitively indicate the modification's location on the amino acid sequence. For example, where the modification is on an amino acid close to the N or C terminus many of the b and y ions respectively will contain the modification.

In these cases, this can be achieved by comparing the amino acid sequences of the matched fragments with the amino acid sequences of fragments without the modification seen in the data. For example, if a b-type ion containing AA1-80 in the protein is seen without the modification, and a b-type ion containing AA1-85 in the protein is seen with the modification then it can be determined that the modification is attached to AA81-85 of the protein sequence. As a further example, if a b-type ion containing AA1-80 is seen without the modification; a further b-type ion containing AA1-100 is seen with the modification and a y-type ion from AA90-130 is seen without the modification, the modification can be located to AA81-89.

*Identifying the presence of internal fragments*

Internal fragments can be identified by way of observing offsets, for example larger offsets which would not normally be associated with a post-translational modification, for example, over approximately 2 kDa. These offsets correspond with the loss of a common or shared peptide from a set of terminal fragment ions. For example, where there is a set of fragments from the N-terminal (e.g. b ions) in the spectrum data, if a peptide is lost from the N-terminal end of these fragments, this common peptide shows as a modification in the data (that is, it shows up as a fragment: the b ion, with an offset (the loss of the peptide fragment ion). In this way, the method not only can identify modifications, but can also identify internal fragments in the data which may otherwise obscure the data; but additionally can lead to the elimination of these internal fragment ions as containing a modification (assuming the internal fragment ion shows as an unmodified internal fragment). Therefore, as for identifying fragments with a modification, once the offset (indicating loss of a common peptide fragment ion) shows as a plurality of matches (the number of matches being the terminal fragment ions minus the common peptide (the offset)), the fragments with this offset can be investigated further, and the fragments with this offset identified and their amino acid sequence determined. This can be done for example by matching terminal fragments with this offset to the observed fragments, and removing the common peptide from the matched terminal fragment sequences before assignment.

**Method of removing false fragments**

Product ion mass spectra data afforded by fragmenting intact or native proteins can be difficult to interpret. Specifically, it can be challenging to confidently identify isotopic envelopes that correspond with intact protein fragment ions. Deconvolution algorithms are used to identify the mass of protein fragment ions in product ion mass spectrum data. However, many of the envelopes identified by these algorithms cannot be reliably distinguished from the surrounding signal. The inventors have identified a method of effectively removing many of these false envelopes from the list of fragment ions whilst ensuring nearly all of the true envelopes are retained.

*Deconvolution algorithms*

Deconvolution is the mathematical conversion of the $m/z$ measurements in the mass spectrum to a list of fragments, their molecular monoisotopic masses, and their abundance. In this way, distinct fragments are discerned from the original signal which contains many overlapping isotopic envelopes.

The use of more than one deconvolution algorithm applied to the intact protein product ion mass spectrum helps in obtaining the maximum number of true peaks from the original spectrum (as different algorithms will produce a different list of peaks due to the different mathematical methods applied to detect envelopes). Examples of deconvolution algorithms are provided in the examples.

*List of fragments*

The list of fragments output from the deconvolution algorithm corresponds with isotopic envelopes in the spectrum. Isotopic envelopes are comprised of a series of regularly-spaced peaks in a mass spectrum which represent a distribution of a charged isotoplogues with the same molecular formula and charge. By removal of false fragments is meant removal of false isotopic envelopes.

*False fragment*

The deconvolution algorithm(s) produce a list of fragment ions. These list(s) contain not only true fragments but also false fragments. These are isotopic envelopes which have been selected and deconvoluted by the deconvolution algorithm but do not represent true product ions from protein fragmentation. That is, they correspond with algorithmic artefacts rather than true fragment ions. These false fragments have some semblance of isotopic distribution therefore it is often difficult to identify that they are false. Hence, the method here provides, for example, a way of using multiple deconvolution algorithms which identify the maximum number of true fragment ions from a product ion mass spectrum. However, it also filters out many of the false fragments that are also detected after such extensive deconvolution.

*Trained machine learning model*

By model is meant a set of algorithms which can classify putative isotopic envelopes as true ions, or false artefacts of the mathematical deconvolution algorithms.

The model is a machine learning model. One or more of the models may be a trained logistic regression model.

One or more of the models may be a gradient boosting model. Other methods for binary classification may also be used. The input variables one or models have been trained on are listed below.

***Method steps for training one or more models:***

*Training data:*

Input variables describing fragment ion isotopic envelopes are derived from deconvoluted product ion mass spectraand include any one or more of the following measurements (calculations) of the isotopic envelope properties:

1) the charge of the ion and optionally an associated measure of charge uncertainty;
2) a goodness-of-fit score, for example a cosine similarity score, Pearson correlation coefficient, Chi-square goodness of fit test statistic, and/or other custom scoring measures;
3) an interference score, which describes the proportion of signal within the span of the fragment ion's isotopic envelope that can be assigned to the fragment ion;
4) the signal-to-noise ratio, where the noise is defined as the modal peak intensity;
5) the number of missing isotopologues from the isotopic envelope;
6) the standard deviation of the mass errors of individual isotopologues; and
7) the Pearson correlation coefficient calculated by correlating the observed isotopologues intensities with those expected for a typical protein fragment ion

The training data may include all the input variables.
Input variable 2: goodness of fit score, is calculated as follows:

$$\text{score} = \frac{\sum_{i=0}^{n} k \cdot y_{theo_i} \times \min\{\frac{y_{exp_i}}{k \cdot y_{theo_i}}, \frac{k \cdot y_{theo_i}}{y_{exp_i}}\}}{\sum_{i=0}^{n} k \cdot y_{theo_i}}$$

After fitting the theoretical envelope by varying k to minimise S

$$S = \sum_{i=0}^{n} ((k \cdot y_{theo_i} - y_{exp_i}) \times y_{theo_i})^2$$

where y_theo is the vector of theoretical isotopologue intensities and y_exp is the vector of observed isotopologues intensities.
Input variable 7: the Pearson correlation coefficient is calculated as the correlation between the scaled y_theo and y_exp vectors.
The other inputs would be readily calculable by the skilled person in the field.

*Training the model:*

The machine learning algorithm is trained to relate the input variables to the labelled outcome: true fragment or false fragment. To generate the labelled outcome, the envelopes in the spectra are labelled, for example labelled manually as either true or false by an expert. However, labelling may be via other means.
Each model undergoes an individualized training process, where hyperparameter optimization is performed to maximise recall.

***Method of using trained models (optionally wherein one or more of the trained models is trained as above):***

*Input:*

The input is data calculated from the spectrum for a plurality of the fragments in the spectrum. The input data comprises calculations of isotopic envelope properties for one or more fragments in the spectrum. The input data calculated from the spectrum may be that as described above - see training data: input variables. These same values (measurements/properties) are calculated for the fragments (fragment envelopes) in the deconvoluted data and then these values are input into at least 2 trained models. More than 2 trained models may be used. The trained models may be trained as described above.

*Output:*

The output of each trained model is a list of putative false fragments from each model.

*Further processing of output of the trained models to achieve peak list:*

If the output of at least 2 of the trained models agrees that the detected envelope corresponds with a false fragment, then the method identifies these fragments as false in the list of fragments from the spectrum. For example, for each fragment in the list of envelopes, the number of methods that calculate a true probability less than 0.5 are counted. If this number is greater than two, the fragment is classified as false.

Once identified as false, the method can then remove these false fragments (fragment envelopes) from the fragment list. This filtered list of fragments can then be used as further input for example to the method of identifying the presence of one or more modifications in a protein. That is, the data comprising masses of fragment ions in claim 1a) may be a filtered list of fragments.

*Computer program and non-transitory media*

By computer program is meant machine readable program instructions. These may be provided on a transitory medium such as a transmission medium or on a non-transitory medium such as a storage medium. Such machine readable instructions (computer program code) may be implemented in a high level procedural or object oriented programming language. However, the program(s) may be implemented in assembly or machine language, if desired. In any case, the language may be a compiled or interpreted language, and combined with hardware implementations. Program instructions may be executed on a single processor or on two or more processors in a distributed manner.

Therefore also included are one or more non-transitory computer readable media storing machine-readable instructions which, when executed, cause one or more processors to perform the method described herein.

*Apparatus*

The processing circuitry may comprise general purpose processor circuitry configured by program code to perform specified processing functions. Alternatively, the processing circuitry may comprise special purpose processing circuitry. Thus, the configuration of the circuitry to perform its specified function may be limited exclusively to hardware, limited exclusively to software, or a combination of hardware modification and software execution.

The above definitions and explanations apply equally to the apparatus, computer program and non-transitory/transitory media. The apparatus is configured to perform the method of identifying modifications and/or the method of removing false fragments.

Throughout the specification, unless the context demands otherwise, the terms 'comprise' or 'include', or variations such as 'comprises' or 'comprising', 'includes' or 'including' will be understood to imply the method or kit includes a stated integer or group of integers, but not the exclusion of any other integer or group of integers.

Each document, reference, patent application or patent cited in this text is expressly incorporated herein in their entirety by reference, which means it should be read and considered by the reader as part of this text. That the document, reference, patent application or patent cited in the text is not repeated in this text is merely for reasons of conciseness. Reference to cited material or information contained in the text should not be understood as a concession that the material or information was part of the common general knowledge or was known in any country.

**Description of the Figures**

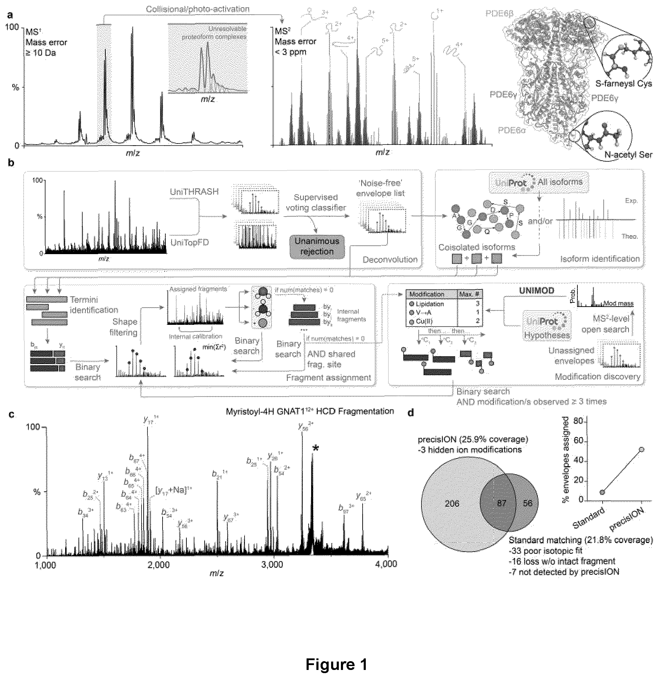

**Figure 1** shows a schematic illustrating a typical native top-down mass spectrometry measurement. Large, unresolvable (by m/z) proteoform complexes can be isolated in the gas phase and activated to form small fragment ions. The mass of each fragment can be very accurately measured, enabling us to confidently identify individual subunits within the complex and the modifications present. Here, all three subunits of bovine PDE6 were identified from bovine rod outer segment membranes. Small modifications, including N-terminal methionine exclusion, N-terminal acetylation, and cysteine farnesylation, that cannot be easily resolved using other structural techniques could be confidently identified using the MS$^2$ spectrum. **B)** Overview of the workflows employed by precisION for spectral deconvolution, protein identification, fragment assignment, and modification discovery. **C)** HCD MS$^2$ spectrum of N-terminally myristoylated GNAT16+ from bovine rod outer segment membranes. Fragments were assigned using precisiON. The most abundant assigned ions are annotated. In total, 52% of the identified envelopes could be assigned as distinct fragmentation products. The asterisk corresponds with the precursor. **D)** Comparison of precisION's performance for the GNAT sample with the typical approach to spectral analysis employed in literature. The Venn diagram displays the number of ions assigned by each algorithm. When using the typical matching approach, many of the identified ions could be disqualified upon manual inspection of the envelope fit, or correspond with unlikely fragmentation products. PrecisION additionally enables fragment ions with unexpected modifications to be observed. The inset shows the difference in the proportion of detected envelopes that could be assigned using each algorithm.

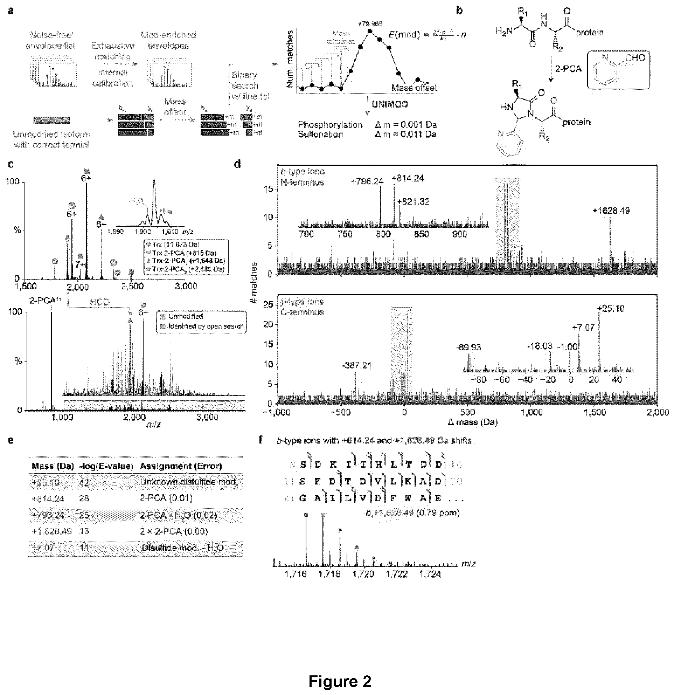

**Figure 2** shows a schematic illustrating the principles of a fragment-level open search. Filtered enveloped lists are first assigned using the exhaustive matching scheme outlined in Figure 1, yielding a list of modification-enriched envelopes. These envelopes are searched for matching sequence ions given the unmodified protein sequence and a mass offset that is applied to each protein terminus. Scanning this mass offset over a large range typically yields several peaks, where the maxima correspond with the masses of fragment ion modifications. The significance of each peak in the offset scan can be calculated using a Poisson distribution, and the identity of each modification can be determined with UniMod. **B)** Reaction of 2-pyridinecarboxaldehyde with the N-terminus of proteins. The label forms a stable cyclic imidazolidinone via an N-terminal imine intermediate. **C)** Native mass spectrum of ~5 $\mu$M) Escherichia coli thioredoxin (Trx) after reaction with 2-PCA-Atto532 (2-PCA, 833 Da) and extensive exchange into 200 mM ammonium actetate (pH 7.0). Thioredoxin with up to three 2-PCA adducts can be observed. The 6+ charge state of the doubly adducted species was isolated (see inset) and subjected to higher-energy collisional dissociation, yielding singly charged 2-PCA, unmodified Trx, and other small fragment ions. After initial assignments (highlighted in green), a large proportion of the spectrum (blue) could be assigned by considering modifications identified in a fragment-level open search. **D)** Offset scan results for the N- and C-terminus of thioredoxin. The most significant peaks for each scan are labelled. **E)** Table of the top five most significant offsets, their statistical significance, and the corresponding modification. Mass errors (Da) are displayed in brackets. **F)** Partial sequence map of Trx showing the position of b-type ions modified with either one (+814.24) or two (+1628.48) 2-PCA molecules. The envelope fit for [b1+2-PCA2]1+ is shown below.

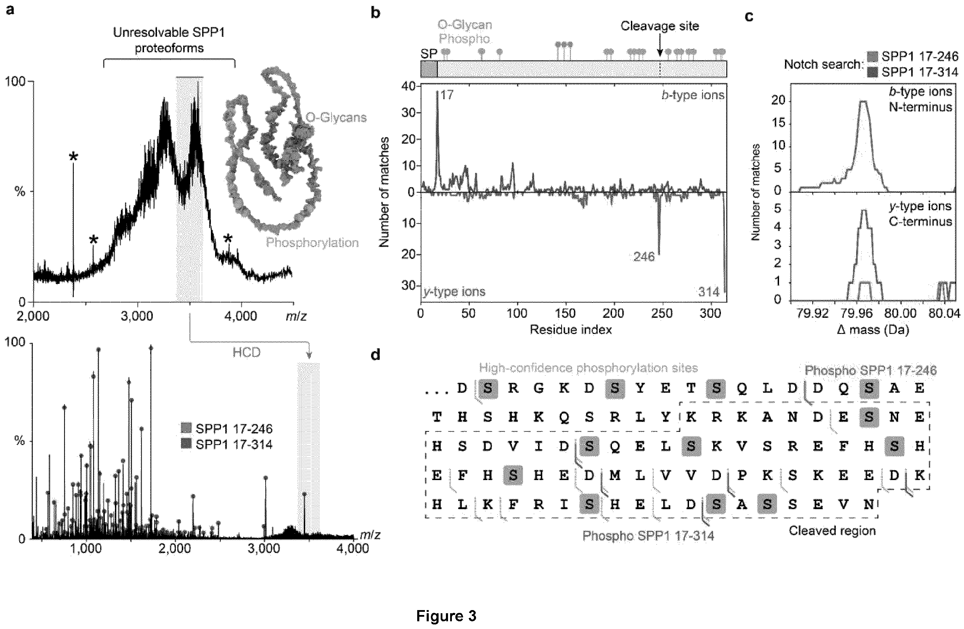

**Figure 3** shows identification of phosphorylated and truncated proteoforms of SPP1 behind complex glycosylation. **A)** Native mass spectrum of SPP1 in 1 M ammonium acetate, pH 7.0. Inset is a potential structure of the intrinsically disordered protein with phosphorylation and glycosylation sites highlighted in blue and pink, respectively. A large region of the spectrum shown in grey was isolated and activated using HCD, producing b- and y-type ions which could be assigned to SPP1. **B**) Multi-notch open search used to identify SPP1 truncations. Truncation sites are annotated next to the peaks. **C)** Fragment-level open search used to identify modifications on each truncated species. The x axis has been expanded to focus on phosphorylation (+79.97 Da).**D)** C-terminal sequence map used to localise phosphorylation sites. Fragments without modification are shown in light grey. Phosphorylated fragments for SPP1 17-246 are shown in pink and phosphorylated fragments for SPP1 17-314 are shown in purple. Potential phosphorylation sites based on literature are highlighted in blue.

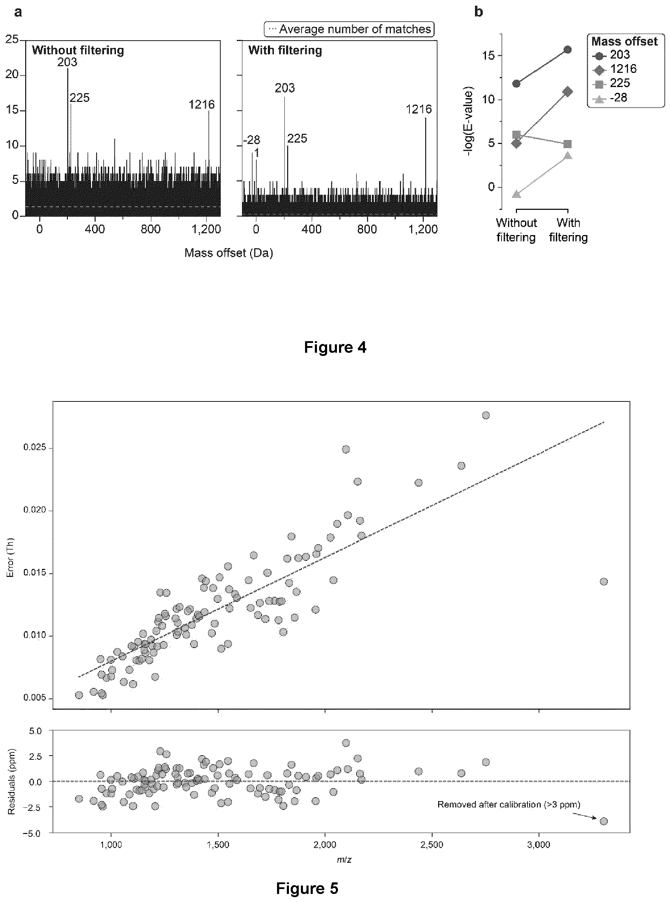

**Figure 4** shows the effect of fragment ion envelope classification on the fragment-level open search. **A)** Fragment-level open search for human ACE conducted without (left) or with (right) pre-filtering of envelopes using the supervised voting classifier. The noise level is reduced following filtering, and only a small number of envelopes matching with significant offsets are lost. **B)** Increase in the significance of offsets with pre-filtering.

**Figure 5** shows linear recalibration using internal calibrants. Example calibration curve showing the reduction in mass errors from >10 ppm to <3 ppm. The calibrated spectrum can be used to perform more sensitive offset searches.

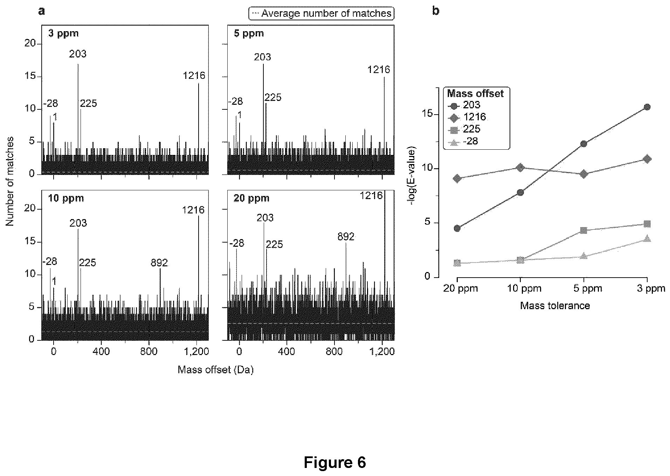

**Figure 6** shows the effect of mass accuracy on the fragment-level open search. a, Fragment-level open search for human ACE conducted at different mass tolerances. The noise level is reduced with tighter tolerances as expected. The resulting mass estimates are also more precise. b, Increase in the significance of offsets with tighter mass tolerances.

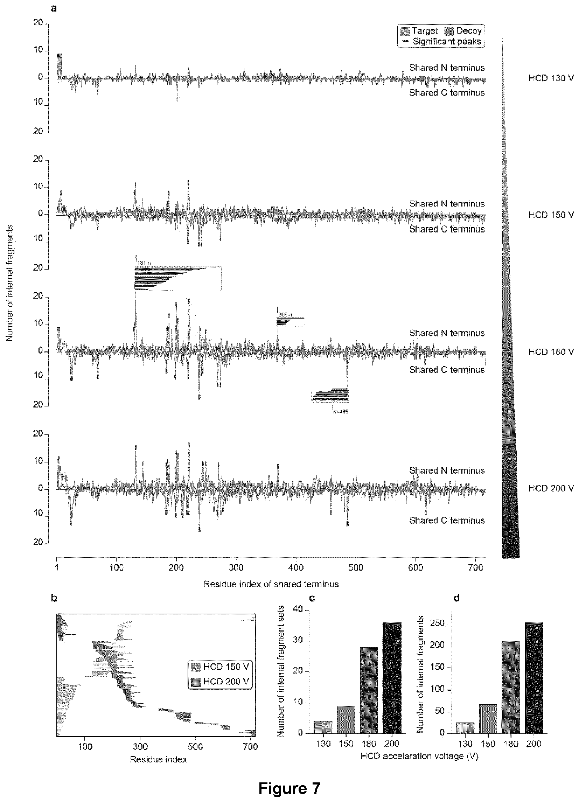

**Figure 7** shows multi-notch fragment-level open search to confidently identify internal fragments. a, Multi-notch offset plot displaying the number of internal fragments with a specific N- (top) or C-terminus (bottom) at HCD acceleration potentials between 130 and 200 V. Sets of internal fragments that contain a statistically significant number of ions are marked in purple. b, Fragment map for human ACE2 at HCD 120 V (blue) and 200 V (purple). Higher acceleration potentials result in the formation of more internal fragments. Internal fragment sets with an E-value of 0.01 were annotated. c, Number of internal fragment sets identified at increasing HCD acceleration potentials. d, Number of internal fragments identified at increasing HCD acceleration potentials.

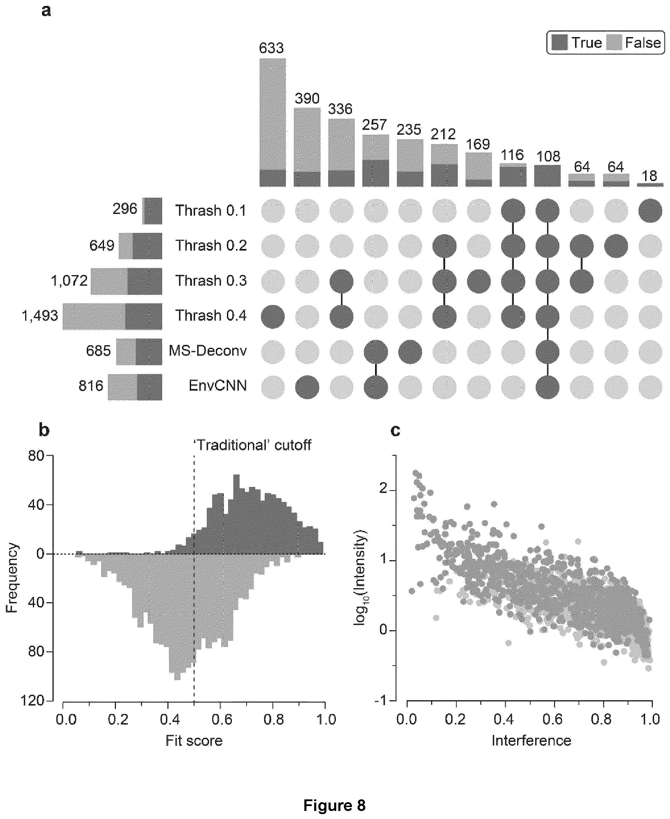

**Figure 8** shows the complexity of isotopic envelopes necessitates multi-dimensional classification. a, Upset plot displaying the overlap between deconvolution algorithms and the number of true and false envelopes within each set (as determined by the supervised voting classifier). Only sets with >15 members are displayed. All algorithms identify unique, real envelopes. Thus, by applying all 6 we ensure that we can exhaustively identify envelopes in the spectrum. As expected, all consensus envelopes are classified as true by our ML algorithm. b, Overlap of fitting scores between true and false envelopes limits the usefulness of a simple single cut-off using fitting score. b, Overlap of true and false envelopes in terms of both abundance/S2N or interference prevents simple cut-offs in these situations as well. In all cases the classification algorithm aligns with our expectations of trends (true = higher fit score, higher abundance, lower interference)

## Examples

Aspects of the present invention will now be illustrated by way of example only and with reference to the following experimentation.

We have designed a novel analytical workflow for the discovery of protein modifications from native top-down mass spectra. We have implemented our approach in precise and accurate Identification Of Native proteoforms (precisION), a data analysis tool and associated GUI specifically designed for the interpretation of complex intact protein fragmentation spectra. By combining state-of-the-art approaches for spectral deconvolution and proteoform identification with a hierarchical fragment assignment workflow that conforms to the principle of parsimony, precisION enables semi-automated and exhaustive spectral interpretation whilst minimising false ion discoveries. Building on such base assignments, precisION additionally facilitates the discovery of hidden protein modifications within the remaining dark matter of fragment spectra, expanding knowledge beyond known proteoform databases.

PrecisION was specifically optimised to overcome the limits of typical nTDMS (native top-down mass spectrometry) datasets. These limitations include: (1) an inability to obtain accurate intact masses of individual proteoform subunits due to sample heterogeneity, protein complex formation, or extensive adduction; (2) less extensive and efficient protein fragmentation due to reduced charging and fragmentation without dissociation; and (3) lower signal-to-noise ratios due to reduced transmission efficiencies and signal dilution (e.g. from extensive neutral losses or adduction). We reasoned that overcoming these challenges would enable us to investigate extensively modified proteins using native MS without experimental intervention - after fragmentation, individual modifications, truncations, and variants can be confidently identified from high mass accuracy (<3 ppm) measurements of small fragments of the protein. These fragments can then be assembled to effectively 'reconstruct' complex intact mass spectra. In principle, this process could enable the observation of small modifications, sequence variants, or truncations within highly heterogenous systems such as protein-lipid complexes, glycoproteins, or polydisperse protein complexes.

PrecisION integrates a number of novel and state-of-the art steps for spectral analysis that can be used separately or within a pipeline, and are designed to enable sensitive, yet accurate, interpretation of complex spectra (Figure 1b).

An important step is that the outputs of multiple modified deconvolution algorithms are clustered and filtered using supervised machine learning (Methods) to eliminate false envelopes that are artefacts of the deconvolution algorithms whilst conservatively retaining envelopes that correspond with true protein fragments. This approach is particularly powerful for the analysis of low signal-to-noise data, where simple scoring measures are often ineffective. The resulting 'noise-free' list of envelopes can then be input into a graph-based de novo sequencing algorithm, or a classical TD-MS database search (MS2 only) to identify protein isoforms present within the activated complex without knowledge of the intact mass. By filtering peak lists prior to searching and assignment, the statistical approaches used to support protein identifications, fragment assignments, and modification discovery (see below) become significantly more powerful as the number of false ion matches is reduced. This greatly increases precisION's sensitivity, even when the total number of observed fragments is low.

Following protein identification, precisION follows a hierarchical ion assignment scheme that was designed to minimise false discoveries by leveraging an empirical understanding of protein fragmentation and the biological mechanisms through which distinct proteoforms are synthesised (Figure 1b). In principle, the algorithm first annotates the ions which are most likely to be observed and removes these from the list of envelopes before assigning less likely products. A complete description of the matching process and underlying rationale is provided in the Methods. This fragment searching to locate offsets which are not the protein sequence is another important step.

Throughout the annotation process, assigned ions are used as internal calibrants, minimising mass errors and enabling high stringency mass tolerances during each successive assignment step. The assignment workflow is semi-automated, ensuring low-confidence fragment matches are manually validated. Together, these steps should ensure exhaustive assignment of fragmentation spectra given an ample understanding of fragmentation ion chemistry and the underlying biology of the system (Figure 1c, d).

**Materials and methods**

**Native top-down mass spectrometry data collection**

Proteins were exchanged into 200-1,000 mM ammonium acetate, pH 7.0 using gel filtration (75 $\mu$L Zeba columns; Thermo Fisher Scientific) and loaded into gold-coated nanoelectrospray ionisation (nESI) emitters fabricated in-house for native MS analysis. Measurements were performed using hybrid quadrupole-linear ion trap-Orbitrap mass spectrometers (Orbitrap Eclipse Tribrid / Orbitrap Ascend Structural Biology Edition Tribrid mass spectrometer; Thermo Fisher Scientific) equipped with static nESI sources. Native protein ions were generated by applying a 0.8-1.2 kV potential to the nESI emitter relative to the instrument's sampling interface (100-200°C) and mild in-source activation (<60 V) was used to enhance ion desolvation. For membrane proteins, more extensive in-source activation (up to 250 V) was required to remove bound detergent molecules. Typically, such high activation offsets also necessitated the application of a compensation potential (~20V) to prevent the transmission of small solvent and detergent clusters. To maximise transmission of large folded protein ions, the instruments were operated in intact protein mode at either standard or high pressure (8 or 20 mtorr $N_2$ in the ion routing multipole/s, respectively). RF amplitudes were maximised to enhance radial trapping of large ions.

MS spectra were acquired in the Orbitrap at a resolution of 17,500-60,000 at $m/z$ 200 with the enhanced Fourier transform enabled. Automatic gain control (target of 4E4-4E5 charges, maximum inject time of 500 ms) was used to control injection times and prevent significant space charging effects. Individual scans were averaged post-transform and, in some cases, subjected to minimal Gaussian smoothing. Data were analysed manually.

For MS spectra, an ensemble of ions within a given $m/z$ window was isolated using either the quadrupole or linear ion trap and subjected to higher-energy collisional dissociation in the ion-routing multipole. To enhance the effectiveness of isolations in the linear ion trap, the isolation waveform was applied for twice the length of time in comparison to standard operating modes. Isolation windows for each dataset are displayed in the main text figures. MS$^2$ spectra were acquired in the Orbitrap at a resolution of 240,000 at $m/z$ 200 with the enhanced Fourier transform enabled and a mass range of $m/z$ 500-4,000. Automatic gain control was again used to minimise space charging effects. For each spectrum, up to 1,000 transients were averaged to increase the signal-to-noise ratio. Spectra were converted to a space-delimited text file format using vendor software (QualBrowser; Thermo Fisher Scientific) and directly processed using precision algorithms.

**Data preprocessing, analysis (PrecisION algorithm) and training the model**

Spectral deconvolution

PrecisION can read profile mass spectra in tab- or space-delimited text file format and has been tested on high resolution Orbitrap (Thermo Fisher Scientific) and FT-ICR data (Bruker). Spectra are deconvolved using one of two user-selected workflows: *Rapid*, or *Extensive.* The rapid approach identifies peaks using a continuous wavelet transformation (CWT), while the extensive approach employs one-dimensional Richardson-Lucy deconvolution. This latter algorithm is similar to UniDec and UniDecNMR, but has been modified to iteratively deconvolve 100 Th windows - using a set of Gaussian point spread functions with varied sigma to account for the $m/z$-dependence of peak width in FT-MS. Richardson-Lucy deconvolution is significantly more computationally intensive than the CWT but demonstrates two key advantages for in-depth spectral analysis: 1) it can more effectively identify overlapping signals in congested regions of the spectrum, enhancing isotopic envelope selection and characterisation; and 2) it is able to reduce some of the effects of noise on peak shape, affording enhanced profile spectra.

Envelope selection and filtering

Envelope selection is undertaken using two approaches - THRASH and TopFD - that differ in their approach to envelope identification. THRASH selects envelopes iteratively from profile spectra, identifying the most abundant potential envelope and evaluating its fit to a theoretical averaging envelope. If the observed envelope satisfies a selected goodness-of-fit threshold, the peaks belonging to this envelope are removed from the spectrum and the algorithm continues. PrecisION utilises an open-source implementation of THRASH implemented in Decon2LS. In the rapid workflow, a single run with a maximum fit score of 0.4 is conducted to minimise processing times. However, due to the iterative nature of the THRASH algorithm, envelopes selected using tighter fitting tolerances are not necessarily subsets of the low-tolerance results. Thus, for the extensive workflow we chose to apply THRASH four times, with fitting tolerances of 0.1, 0.2, 0.3, and 0.4. TopFD builds upon functions from MS-Deconv, implementing a graph-based approach to select the best set of envelopes from a centroided spectrum. Two envelope scoring approaches are available - MS-Deconv and EnvCNN. PrecisION uses both scoring methods to maximise the number of envelopes detected prior to filtering.

The results from both deconvolution algorithms are clustered by mass and a number of qualitative and quantitative features are calculated for each envelope. This includes: 1) the signal-to-noise ratio, where the noise is defined as the modal peak intensity; 2) a fit score; 3) an interference score; 4) the $\chi 2$ goodness of fit test P-value; 5) the Pearson correlation coefficient, comparing the theoretical and observed isotopologue intensities; and 6) the number of missing peaks.

PrecisION's combined deconvolution strategy identifies a large number of envelopes within fragmentation spectra, maximising the number of ions that are included in subsequent searches. However, due to the complex nature of protein fragmentation spectra, these algorithms additionally identify many false envelopes that cannot be reliably distinguished from the surrounding signal. Such envelopes lead to increased noise in *de novo* sequencing, database searches, fragment assignment, and open searches. Thus, we identified the need for an algorithm that can conservatively remove false envelopes without discarding true ions. Our attempts to manually filter envelope lists revealed that defined cut-offs applied to simple scoring measures such as the fitting score, signal-to-noise ratio, or interference score should not be used in classification due to the extensive overlap between real and fake envelopes. Thus, we chose to employ a supervised machine learning algorithm for envelope classification in order to uncover patterns within the complex data. In contrast to previous machine learning-based approaches - which optimised the F1 score and aimed to train general models that could be applied to diverse datasets - here we optimised our model for recall (i.e. minimised the false negative rate) and designed a workflow that allowed models to be easily trained on an individual spectrum or sets of similar spectra. Briefly, a representative set of envelopes are evaluated manually by the user, forming a dataset that is used to train a logistic regression and gradient boosting model. Each model undergoes an individualized training process, where hyperparameter optimization is performed to maximise recall. Once trained, the models are used to evaluate the full set of envelopes in that spectrum. To avoid false negatives, a stringent voting scheme is enacted whereby envelopes are only discarded if both algorithms predict them to be false. This conservative approach lowers the risk of discarding genuine envelopes, which we consider more harmful than maintaining a small population of false positives. As the models are trained on individual spectra, this approach is highly adaptable and can be readily applied to data from different instruments and molecular systems. Furthermore, all trained models can be saved and used to classify related datasets in cases in which there are a number of spectra of similar quality.

Protein identification

Sequence coverage in nTDMS is generally low due to the reduced charge density on precursor ions and the occurrence of bond cleavage without dissociation (e.g. HCnoD). Fragmentation generally occurs at very specific sites, due to either protein sequence (fragmentation often occurs at the C terminus of aspartic acid residues), or structure (disordered and surface-accessible regions, or alpha helices are susceptible). Such minimal and directed fragmentation, in addition to the lack of an intact mass and the presence of unprecedented modifications at protein termini, means protein identification is not always easy in nTDMS.

As part of precisION, we have included two complementary database search algorithms to enable protein-level identifications from nTDMS spectra. Unlike many tools used in denaturing TDMS, we did not expect to identify exact proteoforms in this manner. Instead, specific proteoforms can be detected following protein-level identification using the fragment assignment module. For both algorithms, the user must provide a database file in xml format. Suitable database files are provided for most model organisms and can be constructed from a UniProt database file using ProSight Annotator and some further processing.

The first algorithm utilises a graph-based representation of the spectrum to undertake *de novo* sequencing, identifying sequence tags within the spectrum that can then be matched to specific proteins. This approach is particularly well suited for systems in which fragmentation is driven protons that are localised across specific structural elements of the protein. Long sequence tags (typically >8 residues) can generally provide confident identifications, while shorter tags can be employed to filter putative candidates.

The second algorithm resembles the database search employed in ProSight, generating theoretical fragments (*b/y* or *c/z·* ions) for a library of proteoforms and matching these fragments to the observed data. Unlike ProSight, the library is not filtered by intact mass, equating the algorithm to an open search with an 'unlimited' precursor mass tolerance. Furthermore, to reduce the search space, side chain modifications are not considered during the search. Potential matches are ranked by P-score and reported for manual inspection. Furthermore, to assist in the interpretation of the search results, precisION calculates a native fragmentation propensity score that has been shown to effectively rank proteoform-spectrum matches in nTDMS database searches.

Fragment assignment

Prior to the assignment of fragments, the termini of the isolated proteoforms must be identified. Often termini can be predicted from databases e.g. methionine exclusion, N-terminal acetylation, or signal peptide cleavage. However, in some cases, proteins may be cleaved at unusual sites or modified in complex ways. To detect termini, precisION conducts a fragment-level multinotch search, where notches are selected to correspond with successive single residue cleavages. Notches that lead to a high number of matches likely correspond with the true termini of the proteoforms. To account for N- or C-terminal modifications, users can add defined PTMs to each terminus based on their specific hypotheses.

Once the termini and any fixed modifications have been identified, fragment assignment is conducted in a hierarchical and semi-supervised manner to minimise the likelihood of false discoveries. At collision energies just over the threshold for backbone fragmentation, protonated terminal fragments are typically the most abundant class of ions in intact protein fragmentation spectra. Thus, PrecisION first attempts to assign terminal fragments without variable side chain modifications. Given the tendency for mass analysers to drift, initial matching is typically performed with a low-stringency mass tolerance (e.g. 10 ppm). For all putative assignments (as judged by mass), theoretical ion envelopes are generated using BRAIN and fit to the data. The goodness-of-fit is calculated, and if both the mass error and fitting score are below user-defined thresholds, the ion will be assigned automatically. However, if either threshold is not satisfied, the user is asked to manually evaluate the assignment. As each assignment is made, the matched fragment is removed from the envelope list to prevent repeated matching.

Once the first set of unmodified ions has been assigned, we consider potential variants of these ions that may form through further reaction. Specifically, we calculate the mass of dehydrated, deamidated, and sodiated versions of the assigned ions (using the observed masses to correct for systematic errors in $m/z$), and match fragments to the spectrum in the same semi-supervised manner as described previously. In this case, we can utilise finer matching tolerances (e.g. 3-4 ppm) as systematic errors have been corrected. This cycle iterates multiple times until no further products can be assigned.

Following the assignment of terminal fragments, the spectrum is recalibrated using a linear function to minimise errors in the $m/z$ domain. Recalibration minimises systematic errors and drifts in calibration across the spectrum, ensuring stringent mass thresholds can be used when assigning internal fragments and identifying modifications in later steps. This has been shown to significantly reduce the ion-level false discovery rate.

Finally, we assign internal fragments without variable modifications. The iterative search is conducted as for terminal fragments, but an additional criterion is used for matching to account for the fact that random matches are more likely with internal fragments. Specifically, precisION only assigns internal fragments that belong to a set of internal fragments with a shared N- or C-terminus. Only assigning ions from such a set conforms with the expectation that fragmentation occurs at defined sites in native TDMS and provides additional evidence for any internal fragment assignments. The minimum number of ions that defines a significant set is calculated using a Poisson distribution (see below) that describes the number of shared termini expected for a given offset assuming that there are no true internal fragments present and a threshold expectation value. For degenerate assignments (i.e. observed ions that could be matched to multiple theoretical internal fragments) we follow previously reported strategies that maximise assignment likelihood.

Modification discovery and assignment

Following the assignment of all unmodified ions, the list of unassigned envelopes should be enriched with ions that have been subject to biological (e.g. sequence variants, PTMs) and chemical (e.g. oxidation) modifications. Rather than probing this list using a variable modification search, precisION conducts a fragment-level open search to identify mass offsets that result in a significant number of assignments. Given a range of offsets to consider and a stringent matching tolerance, the algorithm uses a sliding window approach to identify statistically significant mass shifts on either $b$-/$c$-type or $y$-/$z\cdot$-type ions. To calculate probabilities, the null distribution is defined as a Poisson distribution with the expectation of $\lambda$ matches for a given offset, where $\lambda$ is calculated as the mean number of matches in a region of the offset scan where no peaks are observed. Accordingly, the expectation value (E-value) can be calculated using equation 1,

$$\mathrm{E-value} = \frac{\lambda^k e^{-\lambda}}{k!} \times n \qquad (1)$$

where $n$ is the number of matches at a given offset and $k$ is the total number of offsets evaluated during the search. Filtering the envelopes after deconvolution and removing assigned ions before conducting the open search significantly reduces $\lambda$, enabling the method to sensitively detect modified sequence ions within highly complex spectra.

Alternatively, precisiON can also identify enriched mass offsets by searching for common mass differences between unassigned and assigned ions. This can be useful when modifications undergo partial neutral loss or in cases when multiple proteoforms with and without a modification have been isolated.

Once a set of significant mass offsets are identified, these offsets should ideally be assigned to known modifications or variants with defined chemical formulas. Mass errors are typically less than 0.01 Da (post-recalibration), enabling highly confident assignments to be made. Despite this, identifying the exact molecular formula of such modifications can be difficult due to the possibility for multiple or complex modifications. To assist the user in assigning offsets, precisION can map modified ions onto the protein sequence to identify potentially modified residues and directly search UniMod for single modifications and variants that match observations. Alternatively, elemental modification calculators, such as those provided in vendor software (QualBrowser; Thermo Fisher Scientific) can also be used to assist in assignment.

Significant modifications (expressed as chemical formulas or mass shifts) can consequently be assigned to spectra using a variable modification search. To minimise false assignments, we initially consider singly-modified fragment ions using the iterative, semi-supervised framework described above, before moving onto multiple concurrent modifications (Figure 1b). This process will repeat until no more ions can be assigned, at which point, the list of assigned peaks should represent the maximum set of envelopes that can be reliably assigned. An additional fragment-level open search can also be used to confirm this and further filtering can be used to ensure the assigned ions satisfy key metrics e.g. ppm errors.

**Example 1: New method outperforms previous methods**

We compared the algorithm to standard 'forward approaches' to spectral analysis which deconvolve spectra before observed fragments are matched to theoretical sequence ions. This approach is widely used in the freely available programs Prosight Lite (Proteinacious) and MASH Native, as well as the open-source software packages ClipMS, Fragariyo and MS-TAFI. Here, we first deconvolved spectra using THRASH with standard settings and the following parameters: peak background ratio, 3; signal-to-noise threshold, 3; peptide minimum background ratio, 3; minimum charge, 1; maximum charge, 50; maximum mass, 1000000; and maximum fit, 0.4. The resulting list of envelopes was searched for theoretical b- and *y*-type ions as well as their single dehydration / deamidation products and sodiated counterparts using a 10-ppm threshold. A smaller threshold could not be employed due to drifts in the instrument calibration that occur from day to day. Proteoforms that were readily identifiable from the MS spectrum were considered during searching. The resulting matches were used without further processing, in line with the most common practice in the field.

For precisION, data was deconvolved using the extensive workflow with standard parameters. Envelopes were filtered using the supervised voting classifier with at least 100 true and false envelopes used in training. The resulting spectra were assigned using the hierarchical scheme - manual validation was employed for all ions with mass errors greater than 1.5 ppm or fit scores below 0.75. The initial round of matching was performed with a 10-ppm tolerance, with further rounds at 3 ppm. After the initial round of assignments, unexpected modifications were identified using a fragment-level open search and used to conduct a variable modification search as described above. Finally, assigned ions were filtered to remove assignments with mass errors greater than 4 ppm.

To illustrate the utility of precisION for fragment analysis, we reanalysed data from our recent study in which nTDMS was used to characterise the protein landscape within bovine rod outer segments.

**Results:**

Many of the components we identified were found to function in a heterotrimeric G-protein-mediated signalling cascade that is triggered upon rhodopsin activation. When analysing the soluble fraction of the mixture, several charge state distributions were observed, with masses in approximate agreement with the sequence masses of the a and $\beta\gamma$ subunits of a heterotrimeric G-protein, transducin. In the original study, the 6+ charge state of the transducin alpha subunit was isolated and activated using higher energy-collisional dissociation (HCD), affording a plethora of fragment ions that were used to localise the conjugation of a 14:2 fatty acid to the N-terminus (Figure 1c).

Applying precisION to the same dataset, we were able to assign 206 fragment ions that were not detected using standard automated approaches for spectral analysis, increasing the fraction of assigned envelopes over 5-fold (Figure 1d). This included the identification of an alternate 12:0 lauric acid modification on the N-terminus which, upon reanalysis of the MS spectrum, was detected on ~11% of the alpha subunits.

Surprisingly, 56 ions that were assigned using the standard approach were not detected by precisION. However, upon close inspection, many of these assignments could be disregarded due to poor fitting of the isotopic envelope or the identification of dehydrated/deamidated band y-type ions without observation of the intact species. This highlights the ability of the semi-automated, hierarchical approach employed in precisiON to significantly reduce the ion-level false discovery rate whilst unlocking an exhaustive understanding of the fragment ion spectrum.

**Example 2: The fragment-level search method allows sensitive identification of protein modifications**

We reasoned that our combined application of supervised deconvolution and hierarchical spectral assignment should afford a list of envelopes that is enriched with fragment ions that have been chemically or biologically modified (Figure 2a). Thus, by examining the remaining ions following an initial round of assignment, we predicted that fragmentation spectra could be used to sensitively detect protein modifications, even in the absence of an intact proteoform mass. PrecisION identifies modifications using a fragment-level open search, where a variable mass offset is applied to each protein terminus and the number of matching fragments is evaluated as a function of the offset (Figure 2a). Offsets that result in a significant number of matches (E-values calculated from a Poisson distribution) can then be assigned to specific modification/s using UniMod, UniProt and/or elemental composition calculators (Figure 2a).

As expected, applying this algorithm to modification-enriched and filtered envelopes was found to reduce background matching, increasing the significance of true offsets. The method's sensitivity was further boosted by spectral recalibration which allowed for the use of tighter mass tolerances. Recalibration additionally increased the accuracy of mass estimates - for Orbitrap data, errors are typically less than 0.02 Da - enabling confident assignment of modifications.

**Results:**

The results are shown in Figure 2.

To investigate the capabilities of the fragment-level open search, we endeavoured to characterise the products of 2-pyridinecarboxaldehyde (2-PCA) protein labelling. 2-PCA-based reagents specifically label proteins and peptides at their N-terminus (Figure 2b), finding use in proteomics and single-molecule methods. However, their selectivity has been called into question by recent studies that have detected multiple conjugation events but have been unable to localise such additional modifications. We reacted a 2-PCA-Atto532 conjugate (herein referred to as 2-PCA) with *Escherica coli* thioredoxin for 24 hours, then extensively exchanged the modified protein into 200 mM ammonium acetate, pH 7.0. Native mass spectrometry revealed the presence of multiple proteoforms within the resulting mixture with masses of 11,673, 12,488, 13,321, and 14,153 Da (Figure 2c). These complexes were assigned to thioredoxin with 0, 1, 2, or 3 2-PCA adducts, respectively. Interestingly, while the first adduct had a mass of ~814 Da (corresponding with a condensation reaction), further adducts were found to form from mixtures of hydrated (+832 Da) and dehydrated (+814 Da) 2-PCA. This aligns with observations from previous studies, and indicates a potential secondary mode of reactivity or non-covalent association.

To localise these secondary modifications, we isolated and activated the 6+ charge state of the doubly-adducted species and subjected it to higher-energy collisional dissociation. Analysing the resulting fragmentation spectrum with precisION initially detected a number of unmodified *b*- and *y*-type ions, suggesting that 2-PCA is partially labile under these activation conditions (Figure 2c). This is further supported by the detection of a singly-charged ion with *m/z* 329, corresponding with the dissociated tag.

As expected, much of the fragment spectrum remained unassigned without considering protein modifications (Figure 2c). Thus, we turned to a fragment-level open search to identify sets of modified sequence ions. Scanning between -1,000 and 2000 Da revealed several statistically significant mass offsets, each of which could be readily observed above the noise (Figure 2d, e). The top five most significant modifications could be classified into two classes by matching the exact mass to defined chemical formulae (Figure 2e). The first class of modifications consisted of +25.10 and +7.07 (+25.10-$H_2O$) shifts located on *y*-type ions. Mapping these modifications localised them onto the redox-active disulfide bond responsible for thioredoxin activity, but the identity of this PTM has not yet been identified. The second class of modifications corresponded with the addition of 2-PCA onto b-type ions (Figure 2e). Both single (+814.24) and double (+1,624.48) condensations of 2-PCA onto the protein could be observed, with both adducts localised on the N-terminal serine (Figure 2f).

**Example 3: The method can identify specific proteoforms under the mask of glycans which are normally a significant challenge**

Many PTMs have precisely defined masses, enabling their facile detection and quantification by intact protein mass spectrometry. However, glycans pose significant challenges in both native and denaturing TDMS due to their inherent heterogeneity. Glycosylation can extensively dilute protein signals across a heterogenous ensemble of species with distinct masses, preventing resolution of individual species and thereby hindering spectral interpretation. Uncovering aspects of protein structure beneath this glycan 'mask' should enhance our understanding of the interplay between glycosylation and other PTMs, particularly for mammalian proteins - many of which are extensively glycosylated.

We sought to evaluate the utility of nTDMS and precisION for the characterisation of mammalian glycoproteins, focusing on the cytokine and non-collagenous bone protein osteopontin (SPP1). SPP1 is known to undergo a plethora of PTM-sensitive interactions with cell-surface receptors and has been closely related with tumour proliferation, metastasis, and treatment resistance.

**Results:**

The results are shown in Figure 3.

Human SPP1 from HEK293 cells is O-glycosylated, resulting in a complex mass spectrum populated by a diverse range of unresolvable proteoforms (Figure 3a). Due to such heterogeneity, it was challenging to confidently assign accurate charge states in the spectrum, preventing meaningful spectral interpretation. We isolated a broad ensemble of SPP1 proteoforms in the gas phase and activated them using higher-energy collisional dissociation, producing a high-quality $MS^2$ spectrum containing -500 resolvable fragment ions (Figure 3a). Due to its intrinsic disorder, SPP1 can be proteolytically processed during synthesis and trafficking, likely affecting its interactions and function. However, the locations of proteolytic sites are poorly understood and cannot be measured from intact mass measurements alone due to the inherent heterogeneity. We developed a variant of the fragment-level open search that scans across distinct notches to identify protein termini, and found that two major proteolytic products were present in the mixture (Figure 3b). The longer product, SPP1 (17-314), corresponded with the full-length protein after signal peptide cleavage, while the second product, SPP1 (17-246) was further cleaved at the C-terminus between Y246 and K247. This proteolysis site has not been previously identified. Both of the truncated proteoforms are present in the $MS^1$ spectrum but cannot be resolved. However, by analysing the fragments we were able to detect both species, identifying a new site for endogenous proteolysis.

To determine if the SPP1 truncation has any further effects on the protein's modification state, we conducted a fragment-level open search for both species and compared the results. From both these searches we identified both *b*- and *y*-type ions with an additional phosphate group (+79.97 Da), indicating phosphorylation at both protein termini (Figure 3c). This is in line with past literature focused on SPP1, which is known to contain at least 17 high-confidence phosphorylation sites (Figure 3b). Intriguingly, we did not observe any sequence ions modified with 2 or more phosphate groups for either species, suggesting a majority of the potential sites for phosphorylation are unoccupied in our preparation.

The C-terminal truncation we localised removes seven high-confidence phosphorylation sites from the protein (Figure 3b). Thus, we hypothesised that the truncation may decrease overall SPP1 phosphorylation. This hypothesis was supported by the paired open searches, which revealed that fewer phosphorylated ions could be observed for the truncated species than the intact protein (Figure 3c). Mapping modified peptides onto the protein sequence enabled many of the phosphorylation sites for the full-length protein to be localised onto serine residues that are removed upon cleavage (Figure 3d). However, some persistent C-terminal phosphorylation was still retained upon truncation at S234. Taken together, it is likely that such cleavages could modulate SPP1 interactions by removing functional phosphorylated residues.

**Example 4: Filtering synergises with internal fragment level search to increase the significance of off-sets**

The native ACE2 dimer produced in HEK293 GNTI$^{-/-}$ cells was subject to beam-type collisional activation (HCD 130 V) in the front HCD cell of a quadrupole-linear ion trap-Orbitrap (Ascend Structural Biology Edition, Thermo Fisher) hybrid mass spectrometer. The fragments were measured in an Orbitrap mass analyzer and the resulting mass spectrum was afforded by an enhanced Fourier transform. The length of the transient was chosen such that the resolution at a mass-to-charge ratio of 200 was 240,000. Isotopic envelopes were detected using THRASH with scoring cut-offs of 0.1, 0.2, 0.3, and 0.4 as well as TopFD using MS Deconv and EnvCNN scoring methods. The resulting list of envelopes was then optionally (not done for left plot in Figure 4) subjected to classification using the supervised voting classifier described previously. b- and y-type ions were assigned before the dehydrated, deamidated and sodiated counterparts of these assigned ions were also considered. These ions were used as internal calibrants to recalibrate the spectrum. An open search was then conducted with a mass tolerance of 3 ppm to identify significant mass offsets at the N-terminus of the protein. The results from that open search are shown in Figure 4 alongside the significance of the 4 most significant mass offsets. For the data that was classified prior to matching, we observed mass offsets corresponding with CO loss (-28 Da), monoisotopic errors (+1 Da), HexNAc (+203 Da), sodiated HexNAc (+225 Da) and $Hex_5HexNAc_2$ (+1,216 Da).

**Results:**

The results are shown in Figures 4-8.

Figure 4 shows mass offset plots showing the number of fragments matched as a function of the mass offset applied to b-type ions for human ACE2. The offset search was conducted without (left) or with (right) pre-filtering of envelopes using the supervised voting classifier. In both cases, typical b-type ions (i.e. mass offset of 0) and their dehydrated, deamidated and sodiated counterparts were matched before the search. However, the noise level is reduced following filtering. Comparison of the statistical significance of the detected mass offsets with and without filtering shows that filtering increases significance (see Figure 4b).

The plot in Figure 5 shows the effect of recalibration. Figure 5 displays the mass errors of matched fragments as a function of m/z after an initial round of matching fragments to ACE2 with a 15 ppm tolerance. The dotted line represents the line of best fit used to recalibrate the spectrum. The residual plot below show most errors are reduced to <3 ppm. Any assignment that are not within a user defined *tight* tolerance will be deleted following calibration. The application of recalibration allows one to use smaller mass tolerances in the search. In turn, this increases the significance of the match as shown in Figure 6.

Figure 6 shows the mass offset plots showing the number of fragments matched as a function of the mass offset applied to b-type ions for human ACE2 (see Figure 6a). The offset search was conducted with a mass tolerance of 3, 5, 10, or 20 ppm for matching. Tighter tolerances result in less noise in the offset plots, increasing the significance of the identified offsets. Comparison of the statistical significance of the detected mass offsets at each mass tolerance is shown in Figure 6b. As you can see from this figure, tighter tolerances increase significance.

Figure 7 shows the mass offset search using a list of defined mass offsets to identify internal fragments. ACE2 was fragmented using HCD with acceleration voltages of 130, 150, 180 and 200 V and the resulting spectra were subjected to multi-notch mass offset searches, where the size of notches was chosen to correspond with cleavage of successive residues from the N-terminus (upwards facing plots) or the cleavage of successive residues from the C-terminus in addition to the loss of one water (downwards facing plots). These offsets are specifically chosen to allow for the identification of sets of internal fragments with a shared N- or C-terminus. Offsets were defined as significant based on a mathematical description of the noise in the offset plot (corrected Poisson distribution). Significant offsets at each energy are marked with purple lines. These offsets were used to assign sets of internal fragments with a shared terminus that can then be assigned. In b-d, we show that higher collision energies result in the formation of more internal fragment sets, and more internal fragments.

Figure 8a shows an upset plot displaying the overlap between the different deconvolution algorithms used and the number of true and false envelopes within each set (as determined by the supervised voting classifier). Only sets with >15 members are displayed. All algorithms identify unique, real envelopes. Thus, by applying all 6 we ensure that we can exhaustively identify envelopes in the spectrum. However, by using all 6, this does create additional false envelopes which need to be identified and removed from the peak list. All consensus envelopes are classified as true by our ML algorithm.

In figure 8b overlap of fitting scores between true and false envelopes shows the limits of a simple single cut-off using fitting score. Overlap of true and false envelopes in terms of both abundance/S2N or interference prevents simple cut-offs in these situations as well. In all cases the classification algorithm aligns with our expectations of trends (true = higher fit score, higher abundance, lower interference).

**Claims**

1.  A computer-implemented method of identifying the presence of one or more modification(s) of a protein, the method comprising:

    a) obtaining deconvoluted product ion mass spectrum data comprising masses of fragment ions produced by activating intact protein ions;
    b) applying a mass offset to a plurality of theoretical terminal fragment ion masses calculated from the amino acid sequence of the protein, and matching the masses including the offset to the list of observed masses in the deconvoluted product ion mass spectrum data; and
    c) if a plurality of matches are identified in b), identifying the presence of a modification with mass equal to that of the mass offset in a plurality of terminal fragment ions originating from the protein, optionally further comprising
    d) identifying fragments comprising the modification(s) from the plurality of terminal fragment ions in c); and/or identifying the modification(s).

2.  The method of claim 1, wherein:

    a) before step b) of claim 1, masses in the product ion mass spectrum data matching calculated masses based on only the amino acid residues of the fragments are removed, optionally additionally removing masses matching dehydrated, deamidated and alkali metal ions of these calculated masses; and/or
    b) the modification is a covalent modification, optionally a post-translational modification.

3.  The method of any of the preceding claims wherein identifying the fragments comprising the modification(s) comprises determining the amino acid sequence of the modified fragment ions, and optionally wherein the method further comprises localizing the modification(s) to one or more amino acids in the protein by:

a) determining where the determined amino acid sequences of the modified fragment ions overlap; and/or

b) comparing the amino acid sequences of the matched fragments which comprise the modification(s) with amino acid sequences of fragments determined from observed masses in the data corresponding to fragment ions without the modification.

4. The method of any of the preceding claims, additionally comprising identifying the presence of internal fragment ions by identifying an offset corresponding with the loss of a common peptide fragment from a plurality of terminal fragment ions, optionally further comprising determining the amino acid sequences of the internal fragment ions.

5. A computer-implemented method of removing one or more false fragments from a list of fragments generated by one or more mass deconvolution algorithm(s) applied to a product ion mass spectrum produced by activating intact protein ions, the method comprising:

   a) inputting isotopic envelope properties calculated for one or more fragments in a deconvoluted product ion mass spectrum from an intact protein ion into a plurality of trained machine learning models, the plurality of trained machine learning models trained to distinguish between false and true fragments in a list of fragments obtained from the spectrum;

   b) identifying one or more fragments as false if identified as false by at least two trained machine learning models; and

   c) removing the fragment(s) identified as false in b) from the list of fragments.

6. The method of claims 1-4, wherein the product ion mass spectrum data is obtained by performing the method of claim 5 to remove false fragments.

7. The method of:

   a) any of claims 1-4, wherein recalibration of the spectrum is performed before step b) of claim 1 based on matching observed masses in the data with theoretical fragment ion masses calculated from the amino acid sequence of the protein; or

   b) claim 5, wherein recalibration of the spectrum is performed after step c) on the filtered list of fragments without the false fragments based on matching observed masses in the list of fragments with theoretical fragment ion masses calculated from the amino acid sequence of the protein; or

   c) claim 6, wherein recalibration of the spectrum is performed after step c) of claim 5 and before step b) of claim 1.

8. The method of:

   a) any preceding claim, wherein the intact protein product ion mass spectrum comprises product ion data at a mass accuracy better than 50 ppm, preferably better than 5 ppm, most preferably better than 3 ppm; and/or

   b) claim 7, wherein the mass accuracy is better than about 50 ppm, optionally better than about 5 ppm or better than about 3 ppm, after recalibration of the spectrum.

9. The method of any preceding claim, wherein the spectrum or data is a tandem mass spectrum/data (MS$^2$ or MS/MS) or multistage tandem mass spectrum/data (MS").

10. The method of any preceding claim, wherein the data or spectrum is native protein data or a native protein spectrum, produced by activating native protein ions.

11. A computer-implemented method for generating a trained model to detect false fragments from a list of fragments generated by one or more mass deconvolution algorithm(s) applied to a product ion mass spectrum obtained from an intact, optionally a native protein sample, the method comprising:

   a) obtaining measurements from deconvoluted intact or native protein product ion mass spectra, optionally wherein the measurements comprise any one or more of the following:

   i) the fragment ion charge; ii) a goodness-of-fit score that measures the similarity of the observed fragment envelope to that of a typical protein fragment ion envelope; iii) the proportion of signal within the fragment ion envelope's span that can be attributed to the fragment ion itself; iv) the signal-to-noise ratio; v) the number of missing isotopologues; vi) the individual isotopologues mass errors; and vii) the Pearson correlation coefficient calculated by correlating the observed isotopologues intensities with those expected for a typical protein fragment ion;

b) obtaining ground truth data for each of the fragments indicating if the fragment is a true or false fragment; and
c) training a machine learning algorithm to relate a) with b) to produce a trained model, wherein the algorithm is trained to maximize recall.

12. The method of claim 5, wherein one or more of the models is trained using the method of claim 11.

13. A computer-implemented trained model obtained from the method of claim 11.

14. One or more computer-readable storage media or a computer program comprising instructions which, when executed by a computer, cause the computer to carry out the methods according to any of claims 1 to 12.

15. An apparatus comprising:

a)

i) a memory storing 2 or more trained machine learning models; and
ii) processing circuitry communicatively coupled to the memory, the processing circuitry configured to:
run the trained machine learning models to receive as input isotopic envelope properties calculated for one or more fragments in a deconvoluted product ion mass spectrum obtained from an intact, optionally native, protein sample;
identify one or more fragment(s) in a list from the spectrum as false if identified as false by at least two of the trained machine learning models; and
remove the fragment(s) identified as false from the list of fragments to output a filtered list of fragments;

and/or
b)

i) processing circuitry;
ii) a memory communicatively coupled to the processing circuitry, the memory storing instructions executable by the processing circuitry for applying a mass offset to a plurality of theoretical terminal fragment ion masses calculated from the amino acid sequence of a protein, and matching the masses including the offset the list of observed masses in deconvoluted product ion mass spectrum data; identifying if there are a plurality of matches identified; and if a plurality of matches are identified, identifying the presence of a modification with mass equal to the mass offset in a plurality of terminal fragment ions originating from a protein;
the processing circuitry configured to:

execute the instructions to receive as input deconvoluted product ion mass spectrum data comprising masses of fragment ions produced by activating an ion of the intact, optionally native protein, optionally wherein the data is the filtered list of fragment envelopes output from a); and
output an indication of the presence of a modification with mass equal to the mass offset in a plurality of terminal fragment ions originating from the protein; and optionally additionally output: the identity of one or more fragments of the protein which comprise the modification and/or the identity the modification.

Figure 1

Figure 2

Figure 3

Figure 4

Figure 5

Figure 6

Figure 7

Figure 8

Europäisches
Patentamt

European
Patent Office

Office européen
des brevets

# EUROPEAN SEARCH REPORT

**Application Number**

EP 24 19 0466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XIAO KAIJIE ET AL: "Top-down protein identification using isotopic envelope fingerprinting", JOURNAL OF PROTEOMICS, ELSEVIER, AMSTERDAM, NL, vol. 152, 27 October 2016 (2016-10-27), pages 41-47, XP029874583, ISSN: 1874-3919, DOI: 10.1016/J.JPROT.2016.10.010 * p. 47, paragraph spanning left an right column p. 48, left column, 1 p. 48, left column, 2 * | 1-15 | INV. G16B40/10 G01N33/68 G16B40/20 |
| X | SCHACHNER LUIS F ET AL: "Reassembling protein complexes after controlled disassembly by top-down mass spectrometry in native mode", INTERNATIONAL JOURNAL OF MASS SPECTROMETRY, ELSEVIER SCIENCE PUBLISHERS , AMSTERDAM, NL, vol. 465, 27 March 2021 (2021-03-27), XP086569595, ISSN: 1387-3806, DOI: 10.1016/J.IJMS.2021.116591 [retrieved on 2021-03-27] | 1-4, 7-10,14, 15 | |
| Y | * p. 1, Abstract | 6 | |
| A | p. 3, left column, 1 p. 3, Figure 1 and description thereof p. 4, left column, 1 p. 4, left column, 2 p. 4, left column, 3 p. 4, right column, 3 p. 6, left column, 2 p. 9, left column, 2 Supplemental information (SI), p. 4, section "2. Steps to determine the exact mass shift for an unexpected PTM from fragmentation data using PROSIGHT LITE" * -/-- | 5,11-13 | |

**TECHNICAL FIELDS SEARCHED (IPC)**

G16B
G01N

The present search report has been drawn up for all claims

| Place of search | Date of completion of the search | Examiner |
|---|---|---|
| Munich | 10 January 2025 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & Schachner Luis F ET AL: "Reassembling protein complexes after controlled disassembly by top-down mass spectrometry in native mode - Supplemental information", International Journal of Mass Spectrometry, 27 March 2021 (2021-03-27), XP093236193, NL ISSN: 1387-3806, DOI: 10.1016/J.IJMS.2021.116591 Retrieved from the Internet: URL:https://www.sciencedirect.com/science/article/pii/S1387380621000713?via%3Dihub#appsec1> ----- | | |
| X | MCILWAIN SEAN J. ET AL: "Enhancing Top-Down Proteomics Data Analysis by Combining Deconvolution Results through a Machine Learning Strategy", JOURNAL OF THE AMERICAN SOCIETY FOR MASS SPECTROMETRY, vol. 31, no. 5, 30 March 2020 (2020-03-30), pages 1104-1113, XP093236057, US ISSN: 1044-0305, DOI: 10.1021/jasms.0c00035 | 5,8-15 | |
| | | | **TECHNICAL FIELDS SEARCHED (IPC)** |
| Y | * p. 1104, Abstract | 6 | |
| A | p. 1105, right column, 1 p. 1105, right column, 4 p. 1106, right column, 2 p. 1106, right column, 3 p. 1106, Figure 1 and description thereof p. 1106, Table 1 p. 1107, left column, 2 p. 1107, left column, 4 * ----- | 1-4,7 | |
| | -/-- | | |

The present search report has been drawn up for all claims

| Place of search | Date of completion of the search | Examiner |
|---|---|---|
| Munich | 10 January 2025 | Mühlbauer, Max |

EPO FORM 1503 03.82 (P04C01)

Europäisches
Patentamt

European
Patent Office

Office européen
des brevets

**EUROPEAN SEARCH REPORT**

Application Number

EP 24 19 0466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHONG JIANCHENG ET AL: "Proteoform characterization based on top-down mass spectrometry", BRIEFINGS IN BIOINFORMATICS, vol. 22, no. 2, 2 March 2020 (2020-03-02), pages 1729-1750, XP093052468, DOI: 10.1093/bib/bbaa015 Retrieved from the Internet: URL:http://academic.oup.com/bib/article-pdf/22/2/1729/36653905/bbaa015.pdf> * the whole document *<br>----- | 1-15 | |

TECHNICAL FIELDS
SEARCHED      (IPC)

The present search report has been drawn up for all claims

| Place of search | Date of completion of the search | Examiner |
|---|---|---|
| Munich | 10 January 2025 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

**EP 24 19 0466**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

**EP 24 19 0466**

The Search Division considers that the present European patent application does not comply with the requirements of unity of invention and relates to several inventions or groups of inventions, namely:

1. claims: 1-15

   Methods, trained model, computer-readable storage medium, computer program and apparatus for identifying protein modifications in deconvoluted product mass ion spectra in top-down mass spectrometry and removing false positive fragments from such spectra.

1.1. claims: 1-4, 6(completely); 7-10, 14, 15(partially)

   A computer-implemented method, the method comprising:
   a) obtaining deconvoluted product ion mass spectrum data comprising masses of fragment ions produced by activating intact protein ions;
   FURTHER COMPRISING
   [the method being a method] of identifying the presence of one or more modification(s) of a protein
   b) applying a mass offset to a plurality of theoretical terminal fragment ion masses calculated from the amino acid sequence of the protein, and matching the masses including the offset to the list of observed masses in the deconvoluted product ion mass spectrum data; and
   c) if a plurality of matches are identified in b), identifying the presence of a modification with mass equal to that of the mass offset in a plurality of terminal fragment ions originating from the protein, optionally further comprising
   d) identifying fragments comprising the modification(s) from the plurality of terminal fragment ions in c); and/or identifying the modification(s).

1.2. claims: 5, 6, 11-13(completely); 7-10, 14, 15(partially)

   A computer-implemented method [comprising]
   a list of fragments generated by one or more mass deconvolution algorithm(s) applied to a product ion mass spectrum produced by activating intact protein ions, the method comprising:
   FURTHER COMPRISING
   [the method being a method] of removing one or more false fragments from
   a) inputting isotopic envelope properties calculated for one or more fragments in a deconvoluted product ion mass spectrum from an intact protein ion into a plurality of trained machine learning models, the plurality of trained machine learning models trained to distinguish between false and true fragments in a list of fragments obtained from the spectrum;
   b) identifying one or more fragments as false if identified as false by at least two trained machine learning models; and

page 1 of 2

33

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
        c) removing the fragment(s) identified as false in b) from
        the list of fragments.
                    ---
```

```
Please note that all inventions mentioned under item 1, although not
necessarily linked by a common inventive concept, could be searched
without effort justifying an additional fee.
```

page 2 of 2

34

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LYNN et al.** Protein Prospector and Ways of Calculating Expectation Values. *54th ASMS Conference on Mass Spectrometry*, 2006

- Ascend Structural Biology Edition. Thermo Fisher